# EUROPEAN PATENT SPECIFICATION

(11) **EP 4 090 419 B1**
(45) Date of publication and mention of the grant of the patent: **17.09.2025**
(21) Application number: 21704314.0
(22) Date of filing: 15.01.2021
(51) Int. Cl.: A61N 1/40

(54) **DEVICE FOR THE THERAPEUTIC AND AESTHETIC TREATMENT OF AT LEAST PART OF A PATIENT'S BODY VIA RADIO FREQUENCY**
VORRICHTUNG ZUR THERAPEUTISCHEN UND ÄSTHETISCHEN BEHANDLUNG MINDESTENS EINES TEILS EINES KÖRPERS EINES PATIENTEN MITTELS RADIOFREQUENZ
DISPOSITIF DE TRAITEMENT THÉRAPEUTIQUE ET ESTHÉTIQUE D'AU MOINS UNE PARTIE DU CORPS D'UN PATIENT PAR RADIOFRÉQUENCE

(30) Priority: 16.01.2020 IT 202000000760
(43) Date of publication of application: 23.11.2022
(73) Proprietor: Jörgens, Marion, 88085 Langenargen (DE); Biscuso, Ettore, 16155 Genova (GE) (IT); Biscuso, Marco, 16149 Genova (GE) (IT)
(72) Inventor: BISCUSO, Ettore, 16155 Genova (GE) (IT); BISCUSO, Marco, 16149 Genova (GE) (IT)
(74) Representative: Bottino, Giovanni
(86) International application number: PCT/IB2021/050297
(87) International publication number: WO 2021/144754

(56) References cited:
- EP-A2- 0 756 879
- US-A- 5 046 495
- US-A1- 2018 015 294
- US-A1- 2019 111 275

## Description

The present invention relates to a device for both therapeutic and aesthetic treatment through radio frequency comprising means for generating radio frequency signals connected to an application head adapted to transmit the radio frequency waves to at least part of the patient's body.

Preferably, but not exclusively, as will be more evident from some illustrative forms, the device object of the present invention is particularly suitable for the treatment of a patient's hands.

However, it is specified that the device object of the present invention can be applied to any part of the body of one or more patients.

That just described is the common configuration of the devices for aesthetic radio frequency treatments known in the state of the art, in which said means for generating radio frequency signals convey, in the different cutaneous layers of the human body, electromagnetic energy and therefore consequent thermal energy (heat) through the application head.

The radio frequency treatment devices known in the state of the art generally have only one emitting element as the only functional component, thus obtaining a bidirectional emission of the radio frequency, thus dissipating part of the emitted energy which will be directed both to the area of the body to be treated, but also towards the operator, i.e., towards an area which must not be subjected to the treatment. The state-of-the-art devices also use a passive metal plate in contact with the patient's skin. The latter is commonly used in conjunction with the monopolar radio frequency heads currently on the market, causing unpleasant small shocks and consequent discomfort to the patient.

Furthermore, such a solution causes an unwanted overheating of the same head with difficulty for the operator in manoeuvring the latter.

Other treatment devices known in the art try to solve such a problem through the use of bipolar application heads.

Such bipolar heads have a radio frequency signal emitting element and a radio frequency signal reflecting element, i.e., the two active metal poles, visible, located a short distance from each other and both in direct contact with the patient's skin. Such heads, while eliminating the discomfort created by small shocks, have the limit of having a lower depth of action.

Other solutions of body treatment devices with radio frequency waves are described in EP0756879, US2019111275 and US5046495 which treat the use of hyperthermia and include the use of large devices, which use frequencies in the order of GHz, i.e., microwaves.

Large transducers are therefore used: these are applicators with a minimum diameter of 200mm. For these microwave emission and high power applicators, a bag containing a coolant must be used, interposed between the head and the patient, to avoid the risk of possible burns. In addition, the devices described in EP0756879, US2019111275 and US5046495 require a particularly sophisticated control unit. The treatment is carried out absolutely in a static position and the presence of operators is still necessary.

In particular, EP0756879 describes a protection device for all the treatments which use electromagnetic waves, both microwaves and short waves, to be used on any radio frequency device and for all medical, oncological, therapeutic disciplines, etc., and serves to avoid radiation beyond the treatment area, therefore it is a passive element which goes beyond any active therapeutic treatment.

US2019111275 describes a microwave hyperthermia medical device, for the therapeutic treatment of certain diseases. As can be seen from the description of such a document, the described device uses an impressive four electrodes fixed simultaneously around the area to be treated and in an absolutely static position and connected through four cables to an external unit for controlling the microwave emission.

There is no movement to avoid the persistence of the joule effect caused by the hyperthermia in a single point, in order to avoid possible burns.

No head is described in US2019111275 which has all the elements within-contained in a single shell of biocompatible material, small and of limited weight.

The device described in US5046495 also uses hyperthermia technology, through the use of very complex and high-weight equipment. A large space is required to contain the bulk of such equipment, as large as a radiology chamber.

The very high frequency irradiation technique in the order of GHz occurs through a large electrode, having a diameter ranging from 200 to 300 mm. In this case, the patient is totally involved during the treatment, as he must lay on a movable surface which is crossed by a huge arc "C" where two emission heads are mounted, which must necessarily be provided with a bag of conductive-cooling fluid to be placed before the area of the patient to be treated. The movement relative to the device is provided exclusively to position and centre the irradiation head in the portion of the body to be treated. Once positioned, the irradiation will occur absolutely statically, therefore without any movement. Document EP-A-0 756 879 discloses a device for the treatment of the patient's body through radio frequency.

There is therefore a need not satisfied by the devices known in the state of the art to obtain a therapeutic and aesthetic treatment device which has simple constructive features and which allows to solve the disadvantages described above.

The present invention achieves the above objects by obtaining a device as described above, in which the application head consists of an outer casing, the outer casing comprising therein at least one radio frequency signal emitting element, at least one dielectric element and at least one radio frequency signal reflecting element.

A head of particular capacitive design is therefore obtained, adapted to deliver electromagnetic energy generated by the generating means, which can consist of a free sinusoidal oscillator, having the possibility of being able to irradiate electromagnetic waves at frequencies which can be established and agreed based on the depth of action of the aesthetic treatment.

Such a configuration allows the three components essential for the transduction of the radio frequency signal to be inserted inside an outer casing.

The presence inside the head of both the emitting element and the reflecting element, and rendering the use of the passive metal plate in contact with the patient's skin superfluous, resolves the drawbacks related to the generation of shocks described above.

Due to the shielding feature thereof, the reflector allows to obtain a unidirectional emission of the radio frequency signal which prevents the emission of the radio frequency upwards towards the operator, thus exploiting the total emission power of the transmitted energy and without causing excessive overheating of the application head.

Unlike the state-of-the-art systems, which use the patient's body as a dielectric, the application head object of the present invention includes the use of a specific dielectric element, inserted between the emitter and the reflector.

Advantageously, furthermore, the outer casing consists of biocompatible insulating material, such that the application head is totally insulated and no metal part comes into contact with the patient's skin.

In particular, the outer casing consists of two half-shells which can be coupled together, which are made of biocompatible insulating material.

This, as well as the subsequent features which will be described below, allows to obtain a high constructive simplicity of the device, and in particular, of the head object of the present invention.

The constructive simplicity obviously allows to obtain advantages not only related to assembly, but also regarding production costs, as well as limiting any malfunctions or wear related to systems which are excessively complex from a constructive point of view.

Still with the aim of obtaining a head of simple assembly and operating efficiency, preferably the emitting element is positioned inside the outer casing in the direction of the user's body and lower with respect to the reflecting element, while the dielectric element is interposed between the emitting element and the reflecting element.

Connecting means are also provided to the radio frequency signal generating means positioned higher than the reflecting element, the emitting element comprising a first contact pin to said connecting means.

According to a preferred embodiment, the emitting element consists of a discoidal element having the first contact pin centrally positioned and extending in the direction of the connecting means.

In combination with such a configuration, as will be apparent from the illustration of some embodiments, advantageously the dielectric element and the reflecting element consist of discoidal elements, which have a central hole suitable for housing the first pin.

From what has just been described, the device for therapeutic and aesthetic treatments object of the present invention can be used in the manner known in the state of the art, i.e., through a specialized operator who passes the application head on the skin relative to the part of the patient's body to be treated to perform the specific treatment.

Like all treatments performed by operators, the treatment with the device object of the present invention is also subject to variability, i.e., to the experience, capacity and conditions of the operator himself.

For this reason, movement means are provided configured so as to move the application head on the patient's body.

The movement means can be controlled by an operator, who simply sets the movements of the head, performed by the movement means.

This allows to solve the problems related to an operator's lack of manual skill, performing movements controlled by mechanical means.

However, according to a preferred embodiment variant, the movement means consist of automated means, so that the operator only has to set a treatment program, which is automatically performed by the movement means.

The automation of the treatment consequently allows to limit the intervention of the operator.

Preferably the head belonging to the device object of the present invention can be moved throughout the area to be treated and in continuous movement, thus avoiding persistence in a single point.

As will be apparent from the illustration of some embodiments, the device object of the present invention includes the use of conductive gel dispensing means, so as to include an automatic deposition of such gel, without the aid of an operator.

It is evident that such a feature works synergistically with the movement means, as such movement means facilitate the deposition of the conductive gel.

Advantageously, the movement means comprise a control unit, configured so as to control the movements of the application head in an automated manner.

An exclusive robotic support is therefore obtained, governed by a dedicated software which performs the massage function by moving the head itself, effectively replacing the treatment performed by a human operator.

The device object of the present invention therefore exploits the operator's skills to establish the type of treatment to be carried out, however it eliminates the subjective part, avoiding the performance of the treatment being subject to the conditions and capabilities of the operator in moving the head.

As anticipated, one of the peculiarities of the device object of the present invention is to obtain a high constructive simplicity, whereby, in order to maintain the effectiveness of the aesthetic treatment, but to limit the components, the head is moved only along a plane.

Advantageously, therefore, the movement means are configured so as to move the application head along a plane substantially parallel to the application plane of the patient's body.

It is obviously possible to include that the movement means also move the application head along a vertical direction with respect to the application plane.

According to a preferred embodiment, the movement means comprise a first motor aimed at moving the head along a first axis and a second motor aimed at moving said head along a second axis perpendicular to said first axis.

As anticipated, it is possible to include dedicated software, aimed at automatically controlling the movement means.

The control unit therefore comprises at least one storage unit, within which control instructions are stored aimed at setting the movement of the motors according to predetermined sequences.

The movement means may comprise a display unit configured so as to display a virtual model of the body part of the patient to be treated.

Finally, as will be evident from the illustration of some embodiments, the head belonging to the device object of the present invention is anchored to a peculiar support arm so as to be able to treat even very small portions, such as a single finger of a hand and without the aid of an operator, in order to avoid any contact contagions during the preparation of the treatment.

These and other features and advantages of the present invention will become clearer from the following description of some exemplary embodiments illustrated in the attached drawings in which:
figure 1 illustrates a perspective of an exploded view of the application head belonging to the device object of the present invention;
figures 2a to 2c illustrate some side views of the application head belonging to the device object of the present invention;
figure 3 illustrates a detail of the application head belonging to the device object of the present invention;
figures 4a and 4b illustrate two perspective views of a possible embodiment of the movement means belonging to the device object of the present invention.
figures 5a to 5e illustrate a possible embodiment of the connecting arm of the head belonging to the device object of the present invention;
figure 6 illustrates a view of the device object of the present invention;
figures 7a to 7c illustrate three views of the support plane of the user's hand;
figure 8 illustrates an exploded view of a possible embodiment of the device object of the present invention.

**It** is specified that the figures attached to the present patent application show some possible embodiments of the device for aesthetic treatments object of the present invention to better understand its advantages and features described.

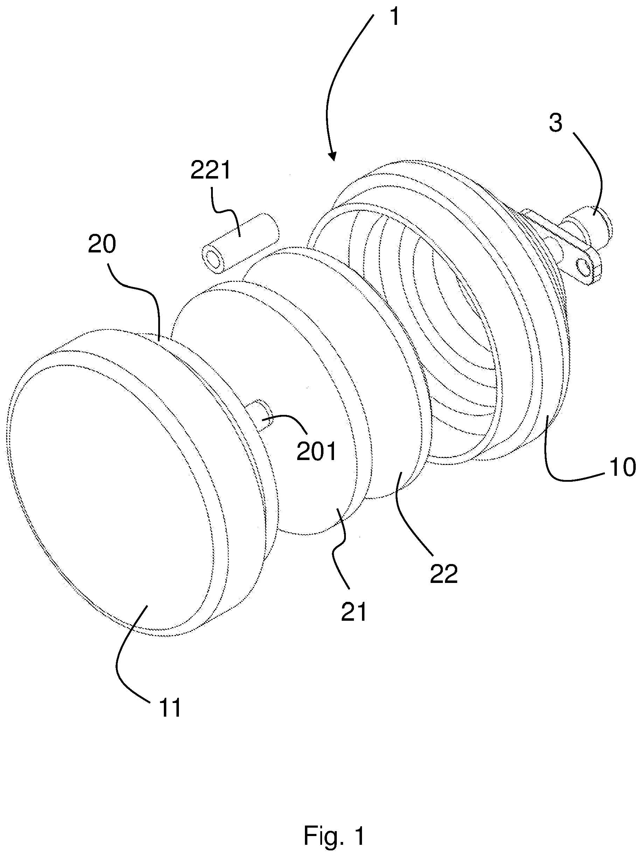

With particular reference to figure 1, a preferred embodiment of the application head belonging to the device object of the present invention is illustrated.

As anticipated, the device object of the present invention comprises means for generating one or more radio frequency signals, which may consist of the common oscillators known in the state of the art, which are connected to an application head which precisely allows the application of radio frequency waves on at least part of the patient's body.

The application head 1 illustrated in figures 1 to 3, comprises an outer casing which consists of two half-shells 10 and 11.

The half-shells 10 and 11 are formed so as to be couplable to each other, according to any one of the methods known in the state of the art, mechanical fastening, chemical or the like.

Once coupled, the two half-shells 10 and 11 identify the outer casing and an inner housing space for housing the remaining components of the head, which will be described later.

Preferably, the two half-shells 10 and 11 consist of biocompatible insulating material, so that the head part which comes into contact with the patient's skin, in this case the bottom wall of the half-shell 11, cannot transmit shocks or currents of any kind to the patient.

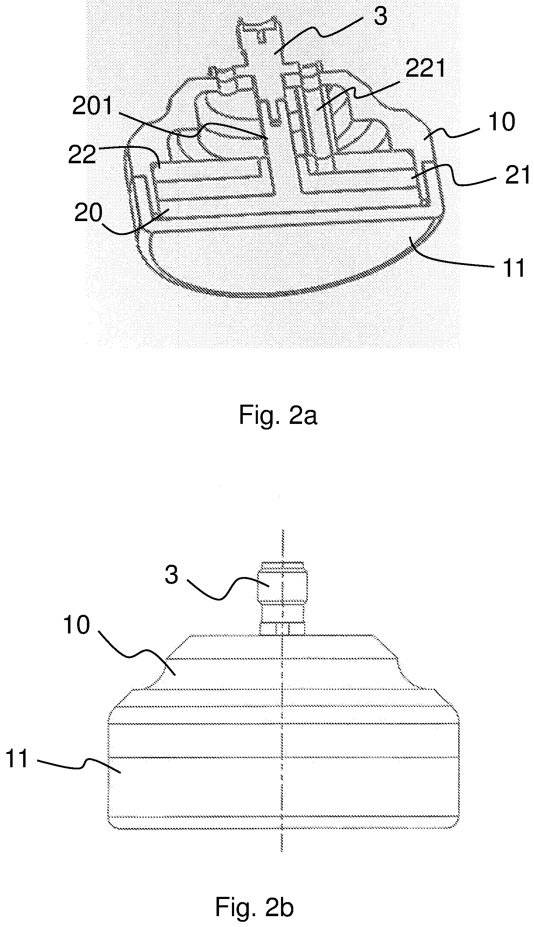

Such a feature is clearly illustrated in figure 2b, in which the half-shell 10 and the half-shell 11 are coupled and define a continuous surface, the outer casing, which encloses all the remaining components of the application head object of the present invention.

The remaining components of the application head 1 are therefore completely contained inside the outer casing and comprise a radio frequency signal emitting element 20, a dielectric element 21 and a radio frequency signal reflecting element 22.

With particular reference to figures 1 and 2a, the emitting element 20 is positioned inside the outer casing in the direction of the user's body and lower than the reflecting element 22.

The dielectric element 21 is interposed between the emitting element 20 and the reflecting element 22.

Preferably the emitting element 20 and the reflecting element 22 consist of aluminium, while the dielectric element 21 consists of vitronite.

The two half-shells 10 and 11 and consequently the outer casing, consist of the insulating material Delrin/Pom.

Furthermore, according to the illustrated variant, connecting means to the radio frequency signal generating means are included above the application head 1.

Such connecting means can consist of a metal connector 3 which is in electrical connection with the emitting element 20.

The metal connector 3 preferably consists of gold.

To allow the connection between the metal connector 3 and the emitting element 20, according to the variant shown in the figures, the element 20 has a metal pin 201, which is in contact, as shown in figure 2a, with the metal connector 3.

Advantageously, the reflecting element 22 must be "grounded", thus it has a second pin 221 grounded to a plate connected to the metal connector 3.

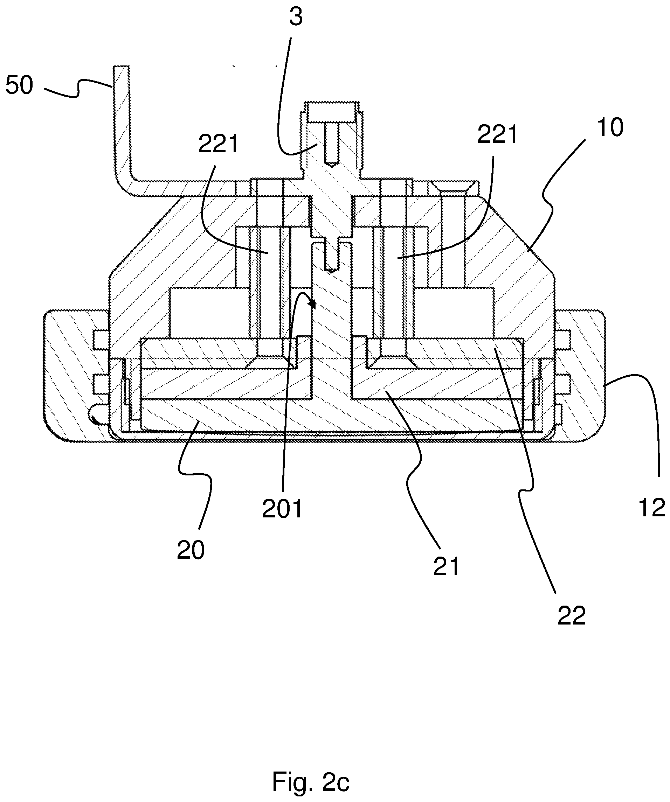

According to the variant shown in figure 2c, the head 1 of the present invention comprises two pins 221 adapted to ground the reflecting element 22.

The variant illustrated in figure 2c further illustrates part of the means for dispensing a conductive gel, in particular a dispensing nozzle 12 which allows to obtain the outflow of the conductive gel in the direction of the body part to be treated.

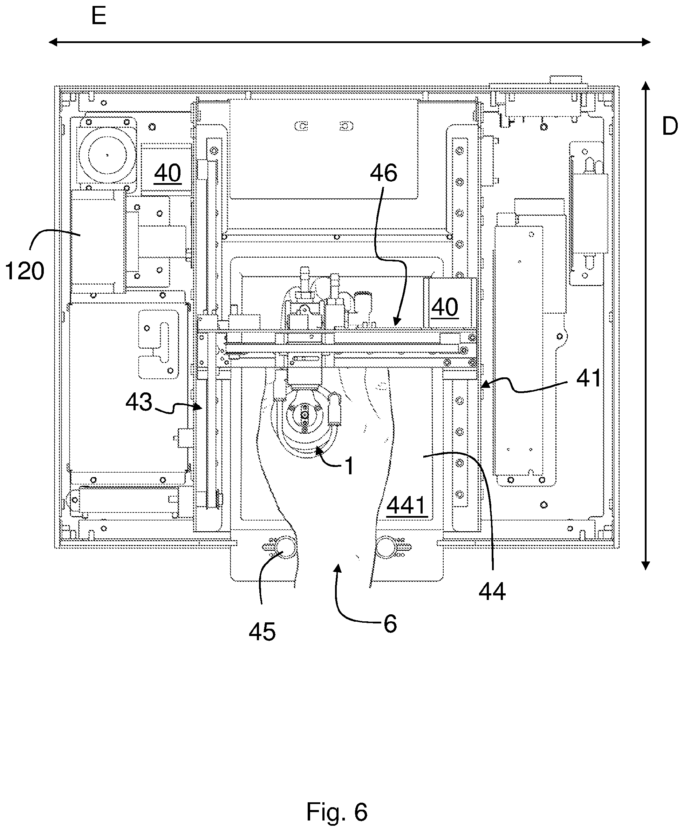

As will be described below, with regard to figure 6, such dispensing means comprise a dispensing nozzle and at least one peristaltic pump 120, fastened to the support structure 4.

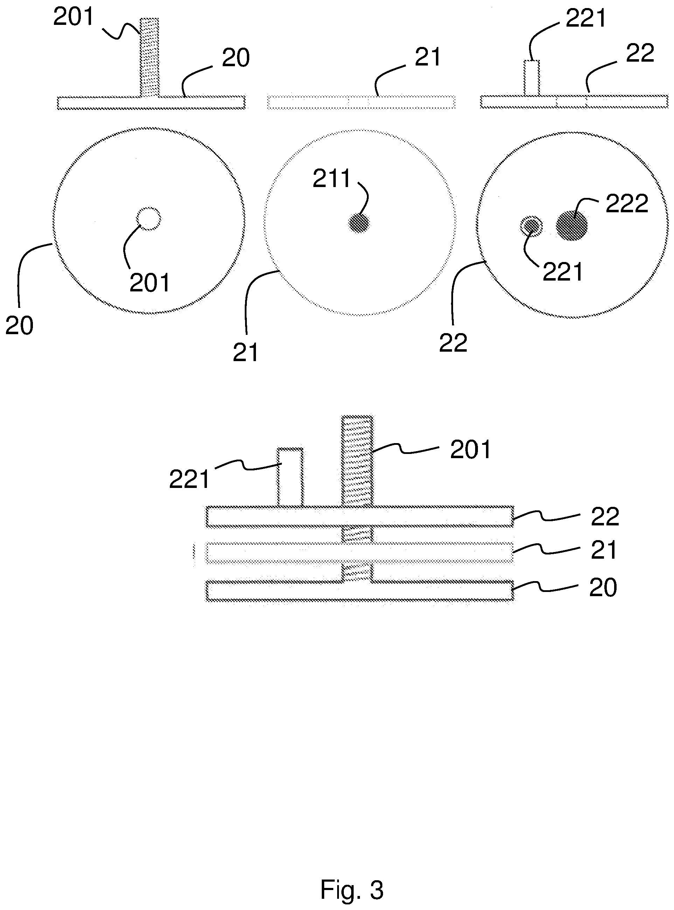

As illustrated in figure 3, to limit the size of the application head 1 and to ensure the correct connection of all the components, the emitting element consists of a discoidal element 20 with the contact pin 201 centrally located and extending in the direction of the connecting means 3, while the dielectric element 21 and the reflecting element 22 consist of discoidal elements, which have a central hole, respectively 211 and 222, suitable for housing the contact pin 201.

The contact pin 221 is instead located eccentrically with respect to the centre of the reflecting element 22.

Figure 3 shows only one pin 221, however it is possible to provide two or more pins 221, as illustrated in figure 2c.

Such a particular configuration allows to obtain the embodiment illustrated below in figure 3, in which the internal elements of the application head 1 are stacked and the pin 201 penetrates the elements 21 and 22 through the central holes 211 and 222.

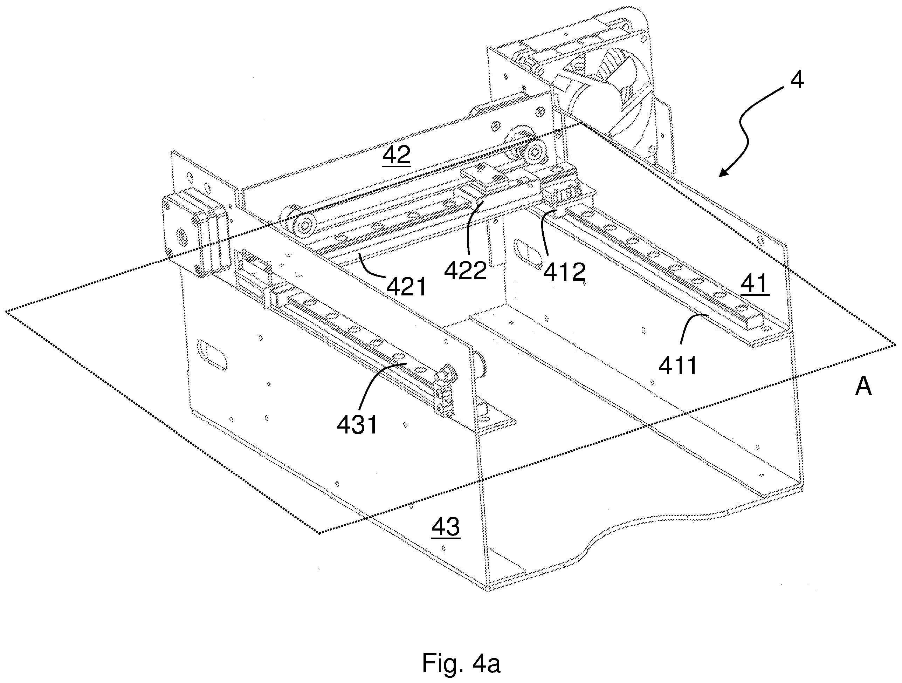

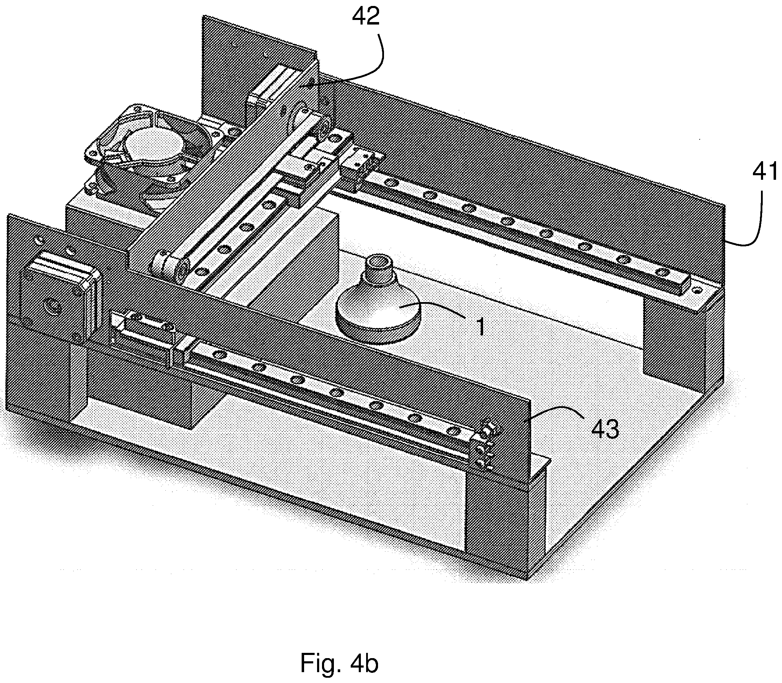

Furthermore, as shown in figures 4a and 4b, the device object of the present invention comprises movement means for the application head 1.

The movement means are preferably obtained by means which allow the application head to be moved automatically on the patient's body.

The example illustrated in the figures refers to the treatment of a hand, but it will become apparent that by changing the dimensions of such movement means, the device object of the present invention can be applied to any part of a patient's body.

The movement means comprise a support structure 4 composed of three walls, so as to define a space therein in which to insert the patient's hand.

The application head 1 is contained within this space, as illustrated in figure 4b.

For illustrative purposes, in figures 4a and 4b, the connection of the head 1 to the movement means is not shown, however it is possible to include a support arm connecting the head 1 and the movement means.

An embodiment of such a support arm will be illustrated and described below.

To move the head 1, preferably two motors, indicated with the number 40 in figure 6, are included which move the head 1 along a plane parallel to the plane of the hand, in particular the plane indicated with the letter A.

The head 1 can be moved through the use of carts which move on rails.

In the particular case of figures 4a and 4b, three side walls 41, 42 and 43 are illustrated, each having a track 411, 421 and 431 on which the carts 412 and 422 slide.

In particular, the cart 412 slides in a longitudinal direction along the track 411, while the cart 422 slides in a transverse direction, perpendicular to the longitudinal axis of the track 411, along the track 421.

Similarly, a cart relative to track 431 may be provided.

It is evident that the combination of the movements of the carts 412 and 422 allows to reach all the points of the plane relative to the treatment area of the patient's hand.

Preferably, two electric motors are included, one for longitudinal movement and one for transverse movement.

Such electric motors are preferably controlled by a control unit, which has processor means for executing a logical program, the execution of which allows instructions to be given to the motors so as to control the head 1 according to predetermined programs.

The logic program is therefore a dedicated software, which allows the operator to select a specific point to be treated or a specific program, to ensure that the treatment occurs completely automatically.

To facilitate the operator's choice, it is possible to provide the movement means with a display unit.

Such a display unit may, in addition to providing the operator with an input/output interface with the device, display a virtual model of the patient's hand (or the body part to be treated), so that the operator selects certain fundamental points for the performance of the treatment.

Preferably the virtual model is not a simple image of any hand, but is the virtual reproduction of the patient's hand, obtained for example through a preventive scan of the patient's hand.

Such a scan allows to find matching reference points between the virtual model and the patient's real hand, so as to provide the device with a specific reference and obtain a precise movement of the application head 1.

Advantageously, the automated movement means are thus governed by CNC software for managing two or more interpolated axes.

The interpolation consists of simultaneously moving two or more axes to reach the final position in the shortest possible time.

In this case the axes allow the head to move according to a predetermined trajectory by the aforementioned software, which contains these trajectories in memory in the form of user-selectable programs.

The movements can be of various types: circular, linear, diagonal, etc. depending on the type of treatment and/or area to be treated. The display unit is preferably touch screen, so as to show a graphical interface of the software itself, displaying a digital representation of a hand, or of the specific hand of the patient, of which the areas to be treated (e.g., fingers, back, knuckles) will be highlighted, according to the program to be selected.

For example, if the palm is selected, the palm of the digital hand will be highlighted, and similarly for the fingers or knuckles.

Advantageously, each program has a predetermined time and cannot be modified by the end user, but only by the programmer in the production step, who reserves the right to modify it where necessary. However, a version may be produced with modifiable time, if requested by the customer.

After selecting the desired program, by touching the area of the hand to be treated on the screen, the operator can start the treatment, so that both the emission of the radio frequency through the head 1 and the movement of the latter are started at the same time.

The remaining time in the treatment step is constantly displayed on the display unit as a digital stopwatch and at the end of the time the software will have a buzzer emit an acoustic signal, which will notify the patient of the end of the treatment.

At this point, the software will command the movement means to move the head 1 to the initial position. During the treatment, a timevarying percentage representing the ON/OFF cycle speed of the radio frequency emission is also present on the display unit.

In particular, 100% represents a continuous RF emission. The percentages programmable in production can range from 0% (corresponding to the absence of RF emissions, but relative to the simple massage of the patient by the head 1) to 100%.

These percentages vary within the total treatment time according to steps with times less than the same and with a minimum of one minute per step.

User-selectable programs, i.e., treatment programs, may also adjust the activation of the peristaltic pump 120, figure 6, so as to adjust the dispensing of the conductive gel through the dispensing nozzle 12 included on the head 1.

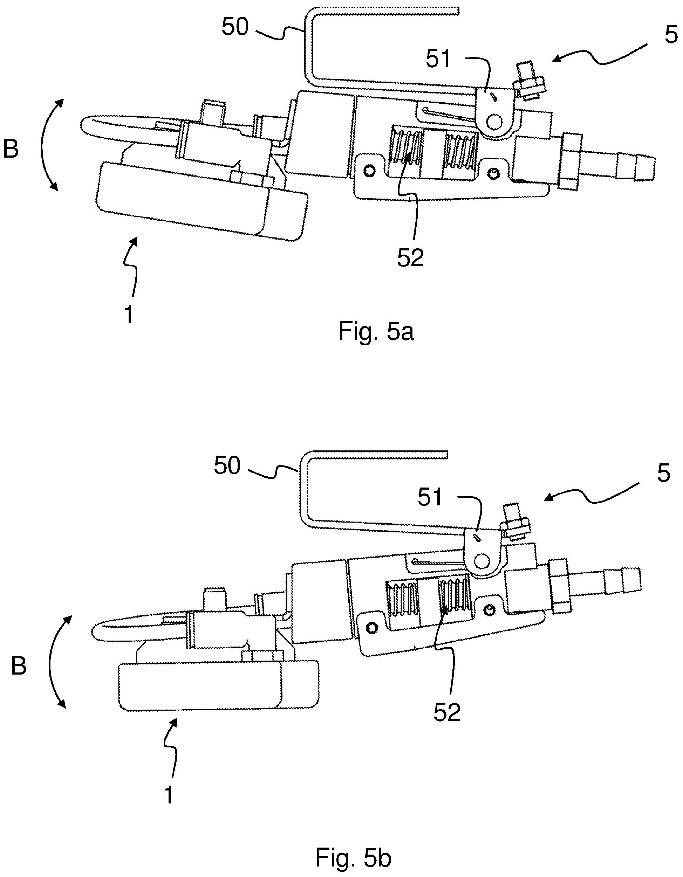

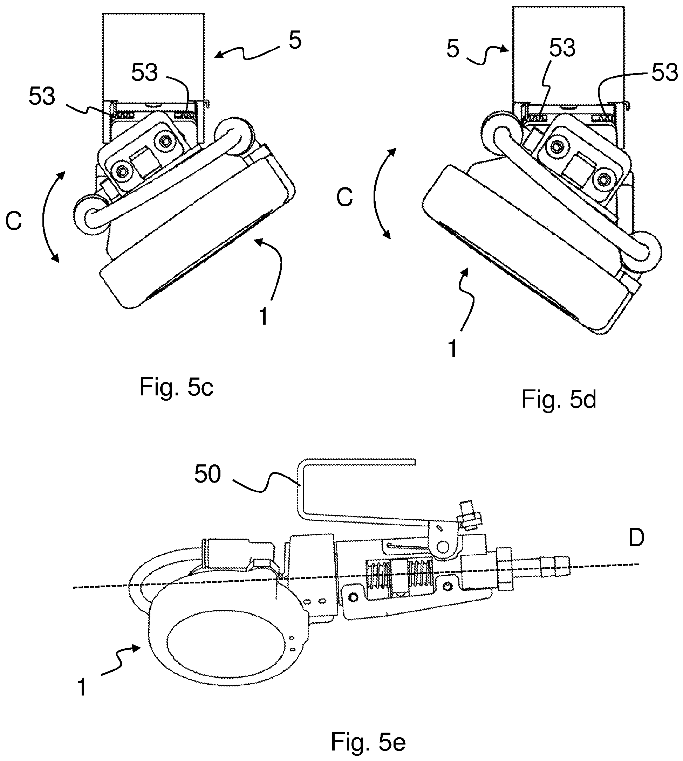

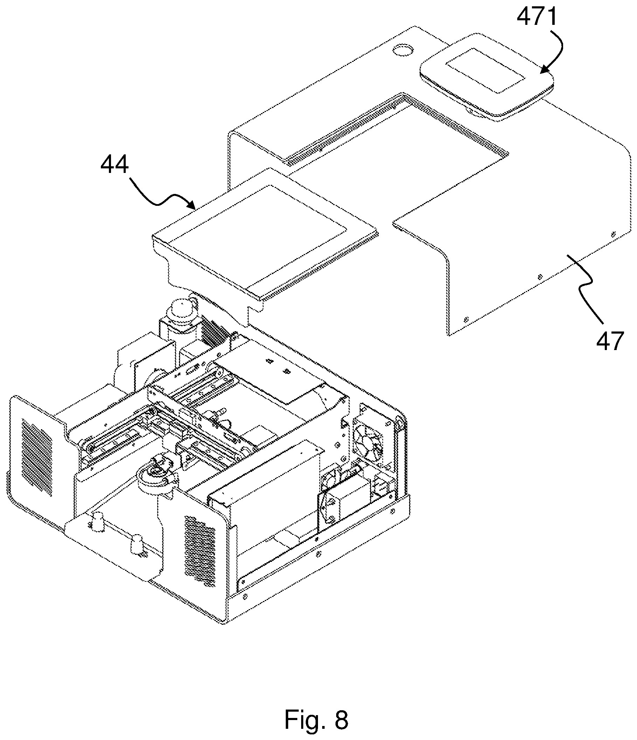

What was just described is further advantageous in combination with the features described in figures 5a to 8.

In particular, figures 5a-5e illustrate a possible embodiment of the connecting arm 5.

The connecting arm 5 has connecting joints to the head 1 which allow at least one degree of freedom of movement of the head 1.

Preferably the head 1 has two oscillation directions, according to the arrow B and according to the arrow C.

In particular, figures 5a and 5b illustrate an oscillation around an axis perpendicular to the longitudinal axis D (figure 5e) of the head 1 and the support arm 5.

The oscillation of the head 1 is passive, i.e., it is caused by the contact of the head 1 with the hand or the part of the body to be treated, i.e., the head 1 can oscillate during the movement of the same, so that the surface thereof adapts to the profile of the part to be treated.

The arm 5 has a fastening bracket 50, which allows the connection to the support structure 4.

Such a support bracket 50 has a joint 51 to which the arm is connected so as to allow the oscillation thereof in the directions of the arrow B.

The head 1 is integral with the arm 5, for which, along the plane perpendicular to the horizontal plane of the device object of the present invention, it moves in a similar way to the support arm 5.

The head 1 then switches from the condition illustrated in figure 5a to the condition of figure 5b and vice versa.

The passage between the two conditions can be obtained thanks to the presence of the force of gravity, which tends to push the head 1 downwards.

Preferably, however, there is a spring 52 which, in equilibrium condition, keeps the head 1 in the condition of figure 5b, so that contact between the body part of the patient to be treated and the head 1 is always ensured.

In addition to the movement shown in figures 5a and 5b, the head 1 can oscillate around the longitudinal axis D of the arm 5, as shown in figures 5c to 5e.

Therefore, the support arm 5 allows the oscillation of the head according to two degrees of freedom, i.e., according to the directions of the arrows B and C.

Also in the case of figures 5c to 5e, there are two springs 53 which permanently keep the head 1 oriented along a horizontal plane, in particular a plane parallel to the horizontal plane of the device object of the present invention.

The bracket 50 of the arm 5 allows the head 1 to be connected to the support structure 4.

In particular, figure 6 shows a top view of the device object of the present invention, according to a possible embodiment.

In this case, a cart 46 is provided which allows the movement of the head 1 along the arrow D.

The bracket 50 is fastened to said cart 46 translatably along said cart, according to the direction indicated by the arrow E.

Thereby, the head 1 can move at all points of the support plane 44 and during such movement, oscillate to adapt to the profile of the hand 6, i.e., of the body part to be treated.

As anticipated, the support structure 4 comprises a peristaltic pump 120 which activates the dispensing of the conductive gel by the head 1 on the hand 6.

The peristaltic pump 1 may be connected to a conductive gel tank and conductive gel nozzle 12.

In particular, the peristaltic pump 120 is connected through a connecting tube of a length such as to allow the movement of the head along the directions of the arrows D and E, without the risk of such a tube detaching from the head 1 or the pump 120.

The device object of the present invention also has a support plane 44, on which the user's hand 6 rests.

The support plane 44 may integrate the display unit 441 aimed at displaying the model of the hand, so as to facilitate the positioning of the hand 6, as described above.

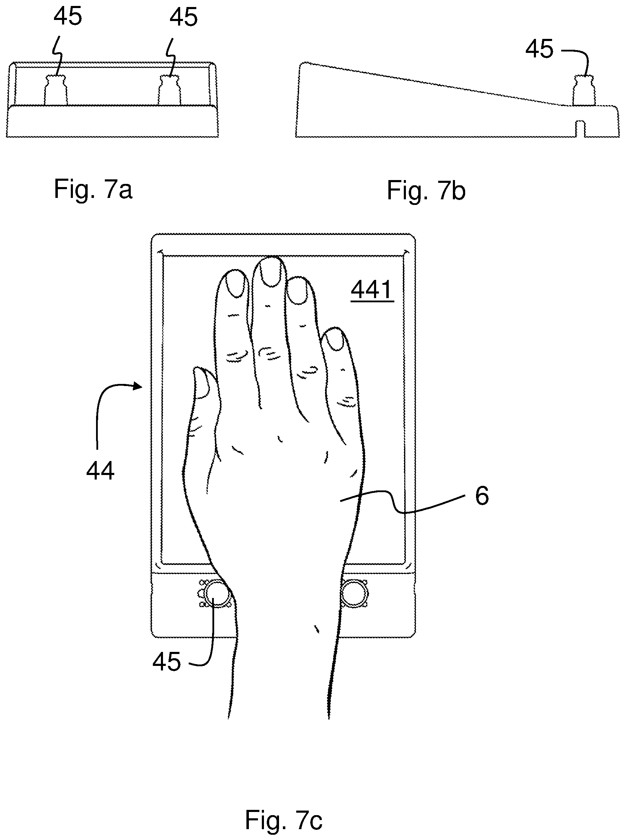

Figures 7a to 7c illustrate a possible embodiment of the support plane 44, which provides means for fixing the hand 6 in position.

Such fixing means consist of sliders 45, mounted translatably on the support plane 44, so as to approach/move away from the wrist of the hand 6 and fix the hand 6 in place.

Finally, figure 9 illustrates an exploded view of the device object of the present invention, in which a casing 47 is illustrated which has an input output unit 471 adapted to allow the user or the operator to select the treatments and/or the time of such treatments, as well as any further parameters.

While the invention is susceptible to various modifications and alternative constructions, some preferred embodiments have been shown in the drawings and described in detail.

It should be understood, however, that there is no intention of limiting the invention to the specific illustrated embodiment but, on the contrary, it aims to cover all the modifications and alternative constructions falling within the scope of the invention as defined in the claims.

## Claims

1. Device for the aesthetic and/or therapeutic treatment of at least part of a patient's body through radio frequency, comprising radio frequency signal generating means connected to an application head (1) adapted to transmit radio frequency waves to at least part of the patient's body,
said application head (1) comprising an outer casing, said outer casing comprising therein at least one radio frequency signal emitting element (20), at least one dielectric element (21) and at least one radio frequency signal reflecting element (22),
wherein
movement means (4) are included configured to move said application head (1) on the body of said patient.

2. Device according to claim 1, wherein said outer casing consists of two half-shells (10, 11) couplable to each other, which half-shells (10, 11) consist of biocompatible insulating material.

3. Device according to claim 1 or claim 2, wherein said emitting element (20) is positioned within said outer casing in the direction of the user's body and below said reflecting element (22),
the dielectric element (21) being interposed between said emitting element (20) and said reflecting element (22),
connecting means (3) being provided to said radio frequency signal generating means located above said reflecting element (22),
said emitting element (20) comprising a first pin (201) in contact with said connecting means (3).

4. Device according to one or more of the preceding claims, wherein said emitting element (20) consists of a discoidal element with said first contact pin (201) centrally located and extending in the direction of said connecting means (3),
said dielectric element (21) and said reflecting element (22) consisting of discoidal elements, which discoidal elements have a central hole (211, 222) suitable for housing said first pin (201).

5. Device according to one or more of the preceding claims, wherein said head (1) comprises at least one fluid dispensing nozzle, which dispensing nozzle is connected to a dispensing pump of said fluid.

6. Device according to claim 1, wherein said movement means (4) are configured to move said application head (1) along a plane (A) substantially parallel to the application plane of the patient's body.

7. Device according to one or more of the preceding claims, wherein said movement means (4) comprise a first motor aimed at moving said head (1) along a first axis and a second motor aimed at moving said head (1) along a second axis perpendicular to said first axis.

8. Device according to one or more of the preceding claims, wherein said movement means (4) comprise a control unit, configured so as to control the movements of said head (1) in an automated manner.

9. Device according to one or more of the preceding claims, wherein said control unit comprises at least one storage unit, within which storage unit control instructions aimed at setting the movement of said motors according to predetermined sequences are stored.

10. Device according to one or more of the preceding claims, wherein said movement means comprise a display unit configured to display a virtual model of the part of the patient's body to be subjected to radio frequency.

11. Device according to one or more of the preceding claims, wherein said head (1) is connected to said movement means through a support arm, said support arm comprising a connecting joint to said head (1), which joint is configured so as to allow the oscillation of said head (1) according to at least one degree of freedom.

12. Device according to claim 11, wherein said support arm is mounted oscillatingly to said movement means.

13. Device according to one or more of the preceding claims, wherein said movement means comprise a support plane of the body part to be treated, which support plane comprises means for fixing said body part in position.

14. Device according to claim 13, wherein said support plane comprises said display unit.

## Patentansprüche

1. Vorrichtung zur ästhetischen und/oder therapeutischen Behandlung von mindestens einem Teil eines Körpers eines Patienten durch Hochfrequenz, umfassend Hochfrequenzsignal-Erzeugungseinrichtungen, die mit einem Applikatorkopf (1) verbunden sind, der angepasst ist, um Hochfrequenzwellen zu mindestens einem Teil des Körpers des Patienten zu übertragen,
der Applikatorkopf (1) umfassend ein äußeres Gehäuse, das äußere Gehäuse umfassend darin mindestens ein Hochfrequenzsignal emittierendes Element (20), mindestens ein dielektrisches Element (21) und mindestens ein Hochfrequenzsignal reflektierendes Element (22), wobei
Bewegungseinrichtungen (4) beinhaltet sind, die konfiguriert sind, um den Applikatorkopf (1) auf dem Körper des Patienten zu bewegen.

2. Vorrichtung nach Anspruch 1, wobei das äußere Gehäuse aus zwei miteinander koppelbaren Halbschalen (10, 11) besteht, wobei die Halbschalen (10, 11) aus biokompatiblem Isoliermaterial bestehen.

3. Vorrichtung nach Anspruch 1 oder Anspruch 2, wobei das emittierende Element (20) innerhalb des äußeren Gehäuses in der Richtung des Körpers des Benutzers und unter dem reflektierenden Element (22) positioniert ist,
das dielektrische Element (21) zwischen das emittierende Element (20) und das reflektierende Element (22) eingefügt ist,
wobei Verbindungseinrichtungen (3) mit den Hochfrequenzsignal-Erzeugungseinrichtungen, die sich über dem reflektierenden Element (22) befinden, bereitgestellt sind,
das emittierende Element (20) umfassend einen ersten Stift (201) in Kontakt mit den Verbindungseinrichtungen (3).

4. Vorrichtung nach einem oder mehreren der vorherigen Ansprüche, wobei das emittierende Element (20) aus einem scheibenförmigen Element besteht, wobei sich der erste Kontaktstift (201) mittig befindet und sich in Richtung der Verbindungseinrichtungen (3) erstreckt,
wobei das dielektrische Element (21) und das reflektierende Element (22) aus scheibenförmigen Elementen bestehen, wobei die scheibenförmigen Elemente ein mittleres Loch (211, 222) aufweisen, das zum Aufnehmen des ersten Stifts (201) geeignet ist.

5. Vorrichtung nach einem oder mehreren der vorherigen Ansprüche, wobei der Kopf (1) mindestens eine Fluidabgabedüse umfasst, wobei die Abgabedüse mit einer Abgabepumpe des Fluids verbunden ist.

6. Vorrichtung nach Anspruch 1, wobei die Bewegungseinrichtungen (4) konfiguriert sind, um den Applikatorkopf (1) entlang einer Ebene (A) im Wesentlichen parallel zu der Applikationsebene des Körpers des Patienten zu bewegen.

7. Vorrichtung nach einem oder mehreren der vorherigen Ansprüche, wobei die Bewegungseinrichtungen (4) einen ersten Motor, der darauf abzielt, den Kopf (1) entlang einer ersten Achse zu bewegen, und einen zweiten Motor, der darauf abzielt, den Kopf (1) entlang einer zweiten Achse senkrecht zu der ersten Achse zu bewegen, umfassen.

8. Vorrichtung nach einem oder mehreren der vorherigen Ansprüche, wobei die Bewegungseinrichtungen (4) eine Steuereinheit umfassen, die konfiguriert ist, um die Bewegungen des Kopfs (1) auf automatisierte Weise zu steuern.

9. Vorrichtung nach einem oder mehreren der vorherigen Ansprüche, wobei die Steuereinheit mindestens eine Speichereinheit umfasst, wobei in der Speichereinheit Steuerungsanweisungen gespeichert sind, die darauf abzielen, die Bewegung der Motoren gemäß vorbestimmten Sequenzen einzustellen.

10. Vorrichtung nach einem oder mehreren der vorherigen Ansprüche, wobei die Bewegungseinrichtungen eine Anzeigeeinheit umfassen, die konfiguriert ist, um ein virtuelles Modell des Teils des Körpers des Patienten, der Hochfrequenz ausgesetzt werden soll, anzuzeigen.

11. Vorrichtung nach einem oder mehreren der vorherigen Ansprüche, wobei der Kopf (1) über einen Stützarm mit den Bewegungseinrichtungen verbunden ist, wobei der Stützarm ein Verbindungsgelenk mit dem Kopf (1) umfasst, wobei das Gelenk konfiguriert ist, um die Oszillation des Kopfs (1) gemäß mindestens einem Freiheitsgrad zu ermöglichen.

12. Vorrichtung nach Anspruch 11, wobei der Stützarm oszillierend an den Bewegungseinrichtungen montiert ist.

13. Vorrichtung nach einem oder mehreren der vorherigen Ansprüche, wobei die Bewegungseinrichtungen eine Stützebene des zu behandelnden Körperteils umfassen, wobei die Stützebene Einrichtungen zum Fixieren des Körperteils in seiner Position umfasst.

14. Vorrichtung nach Anspruch 13, wobei die Stützebene die Anzeigeeinheit umfasst.

## Revendications

1. Dispositif de traitement esthétique et/ou thérapeutique d'au moins une partie du corps d'un patient par radiofréquence, comprenant des moyens de génération de signaux de radiofréquence reliés à une tête (1) d'application adaptée pour transmettre des ondes de radiofréquence à au moins une partie du corps du patient,
ladite tête (1) d'application comprenant un boîtier externe, ledit boîtier externe comprenant à l'intérieur au moins un élément émetteur (20) de signaux de radiofréquence, au moins un élément diélectrique (21) et au moins un élément réflecteur (22) de signaux de radiofréquence,
dans lequel
des moyens de déplacement (4) sont inclus, configurés pour déplacer ladite tête (1) d'application sur le corps dudit patient.

2. Dispositif selon la revendication 1, dans lequel ledit boîtier externe est constitué de deux demi-coques (10, 11) pouvant être couplées l'une à l'autre, lesquelles demi-coques (10, 11) sont constituées en un matériau isolant biocompatible.

3. Dispositif selon la revendication 1 ou la revendication 2, dans lequel ledit élément émetteur (20) est positionné à l'intérieur dudit boîtier externe dans la direction du corps de l'utilisateur et en dessous dudit élément réflecteur (22),
l'élément diélectrique (21) étant interposé entre ledit élément émetteur (20) et ledit élément réflecteur (22),
des moyens de liaison (3) étant fournis auxdits moyens de génération de signaux de radiofréquence situés au-dessus dudit élément réflecteur (22), ledit élément émetteur (20) comprenant une première broche (201) en contact avec lesdits moyens de liaison (3).

4. Dispositif selon l'une ou plusieurs des revendications précédentes, dans lequel ledit élément émetteur (20) est constitué d'un élément discoïdal avec ladite première broche (201) de contact située au centre et s'étendant dans la direction desdits moyens de liaison (3),
ledit élément diélectrique (21) et ledit élément réflecteur (22) étant constitués d'éléments discoïdaux, lesquels éléments discoïdaux présentent un trou central (211, 222) approprié pour loger ladite première broche (201).

5. Dispositif selon l'une ou plusieurs des revendications précédentes, dans lequel ladite tête (1) comprend au moins une buse de distribution de fluide, laquelle buse de distribution est reliée à une pompe de distribution dudit fluide.

6. Dispositif selon la revendication 1, dans lequel lesdits moyens de déplacement (4) sont configurés pour déplacer ladite tête (1) d'application le long d'un plan (A) sensiblement parallèle au plan d'application du corps du patient.

7. Dispositif selon l'une ou plusieurs des revendications précédentes, dans lequel lesdits moyens de déplacement (4) comprennent un premier moteur destiné à déplacer ladite tête (1) le long d'un premier axe et un second moteur destiné à déplacer ladite tête (1) le long d'un second axe perpendiculaire audit premier axe.

8. Dispositif selon l'une ou plusieurs des revendications précédentes, dans lequel lesdits moyens de déplacement (4) comprennent une unité de commande, configurée de manière à commander les déplacements de ladite tête (1) d'une manière automatisée.

9. Dispositif selon l'une ou plusieurs des revendications précédentes, dans lequel ladite unité de commande comprend au moins une unité de stockage, dans laquelle sont stockées des instructions de commande d'unité de stockage visant à régler le déplacement desdits moteurs selon des séquences prédéterminées.

10. Dispositif selon l'une ou plusieurs des revendications précédentes, dans lequel lesdits moyens de déplacement comprennent une unité d'affichage configurée pour afficher un modèle virtuel de la partie du corps du patient à soumettre à une radiofréquence.

11. Dispositif selon une ou plusieurs des revendications précédentes, dans lequel ladite tête (1) est reliée auxdits moyens de déplacement par l'intermédiaire d'un bras de support, ledit bras de support comprenant une articulation de liaison à ladite tête (1), laquelle articulation est configurée de manière à permettre l'oscillation de ladite tête (1) selon au moins un degré de liberté.

12. Dispositif selon la revendication 11, dans lequel ledit bras de support est monté de manière oscillante sur lesdits moyens de déplacement.

13. Dispositif selon l'une ou plusieurs des revendications précédentes, dans lequel lesdits moyens de déplacement comprennent un plan de support de la partie du corps à traiter, lequel plan de support comprend des moyens pour fixer ladite partie du corps en position.

14. Dispositif selon la revendication 13, dans lequel ledit plan de support comprend ladite unité d'affichage.
